# EUROPÄISCHE PATENTSCHRIFT

(11) **EP 4 572 683 B1**
(45) Veröffentlichungstag und Bekanntmachung des Hinweises auf die Patenterteilung: **24.12.2025**
(21) Anmeldenummer: 23761051.4
(22) Anmeldetag: 09.08.2023
(51) Int. Cl.: A61B 17/062, A61B 17/29, A61B 34/00, A61B 17/00, A61B 90/00

(54) **LAPAROSKOPISCHES OPERATIONSWERKZEUG ALS NADELHALTER**
LAPAROSCOPIC SURGICAL TOOL IN THE FORM OF A NEEDLE HOLDER
INSTRUMENT CHIRURGICAL LAPAROSCOPIQUE SOUS LA FORME D'UN PORTE-AIGUILLE

(30) Priorität: 17.08.2022 DE 102022120725
(43) Veröffentlichungstag der Anmeldung: 25.06.2025
(73) Patentinhaber: Universität Rostock Körperschaft des Öffentlichen Rechts, 18055 Rostock (DE)
(72) Erfinder: GROß, Justus, 24235 Lutterbek (DE); PÜSCHEL, Anja, 18055 Rostock (DE); HOHLFELD, Dennis, 18107 Elmenhorst (DE)
(74) Vertreter: Hansen, Jochen
(86) Internationale Anmeldenummer: PCT/DE2023/100583
(87) Internationale Veröffentlichungsnummer: WO 2024/037688

(56) Entgegenhaltungen:
- CA-A1- 3 073 355
- GB-A- 2 599 101
- US-A1- 2009 157 092
- US-A1- 2020 384 643

## Beschreibung

Die Erfindung betrifft einen Nadelhalter zum Halten eines Nahtfadens für roboterassistierte laparoskopische Operationen, die mittels eines Operationsroboters mit wenigstens einem Roboterarm durchgeführt werden, mit einem Montagekopf zum Ankoppeln an den Roboterarm, einem Schaftelement, einem am distalen Ende des Schaftelements angeordneten Gelenkkopf und einem am Gelenkkopf um wenigstens zwei Achsen mittels eines ersten und zweiten Antriebsmittels kippbar angeordneten Zangengreifer, wobei die Antriebsmittel über Umlenkrollen geführte Seilzüge sind, wobei Kraftdetektoren (3) im Nadelhalter (1) angeordnet sind, die die Spannkraft des vom Zangengreifer (14) gehaltenen Nahtfadens (S) erfassen.

Ein derartiges laparoskopisches Operationswerkzeug ist aus der CA 3 073 355 A1 in Form eines chirurgischen Robotersystems bekannt, umfassend ein chirurgisches Roboterinstrument, das einen Endeffektor an einem distalen Ende aufweist, der Endeffektor umfassend zwei gegenüberliegende Backen, die jeweils durch ein Aktuatorpaar betätigt werden, und ein antagonistisches Kabelpaar, die Kräfte ausüben, wenn sie durch das Paar Aktuatoren gespannt werden; und wobei das System umfasst: eine Steuerung, umfassend einen oder mehrere Prozessoren, die mit dem chirurgischen Roboterinstrument gekoppelt sind, wobei die Prozessoren konfiguriert sind zum Empfangen einer Eingabe von einem Eingabemodul zum Bewirken eines gewünschten Zustands des Endeffektors, wobei die Eingabe mindestens einen Nickwinkel und einen Gierwinkel des Endeffektors und einen Backenwinkel zwischen den zwei Backen einschließt; Messen der Positionen der Aktuatorpaare unter Verwendung von Sensoren an den Aktuatoreinheiten; Messen der Spannkräfte an den antagonistischen Kabelpaaren unter Verwendung von Sensoren; Bestimmen einer gewünschten Position des Endeffektors basierend auf dem Nickwinkel und dem Gierwinkel und einer gewünschten Greifkraft zwischen den zwei Backen basierend auf dem Backenwinkel; Erzeugen eines ersten Antriebsbefehls für jeden Aktuator der Aktuatorpaare basierend auf der gewünschten Position und den gemessenen Positionen und Spannkräften; Schätzen einer aktuellen Greifkraft zwischen den zwei gegenüberliegenden Backen basierend auf den gemessenen Spannkräften an den antagonistischen Kabelpaaren; Erzeugen eines zweiten Antriebsbefehls für jeden Aktuator der Aktuatorpaare basierend auf einer Differenz zwischen der gewünschten Greifkraft und der geschätzten aktuellen Greifkraft; und Antreiben der Aktuatorpaare, um die gewünschte Position und die gewünschte Greifkraft zu bewirken, unter Verwendung eines zusammengesetzten Antriebsbefehls basierend auf einer Summierung des ersten Antriebsbefehls und des zweiten Antriebsbefehls. Dabei scheint der hierbei beschriebene Weg zur Greifkraftbestimmung sehr aufwendig und fehleranfällig.

Ferner ist in der US 2020/384643 A1 ein rechnergestütztes Verfahren zur Schätzung der Gelenkreibung in einem Gelenk eines Roboterhandgelenks eines Endeffektors beschrieben. Darin werden sensorische Messungen der Kraft oder des Drehmoments in einem Getriebe, das ein Roboterhandgelenk mechanisch mit einem Aktuator koppelt, durchgeführt. Dazu wird die Gelenkreibung in einem Gelenk des Roboterhandgelenks, das durch den Aktuator angetrieben wird, berechnet, indem die Sensormessungen von Kraft oder Drehmoment auf einen mathematischen Ausdruck angewendet werden, der die Kraft- oder Drehmomentvariablen der Übertragung mit einer Gelenkreibungsvariablen in Beziehung setzt. Ein Nachführfehler des Endeffektors wird ebenfalls berechnet, wobei ein mathematischer Ausdruck in geschlossener Form verwendet wird, der die Gelenkreibungsvariable mit dem Nachführfehler in Beziehung setzt. Auch diese Auswertemethode erscheint rechenaufwendig und abhängig von mathematischen Annäherungen, die wiederum Fehler enthalten können.

Weiter ist in der GB 2599101 A ein chirurgisches Roboterinstrument beschrieben mit: einem Schaft; einem Endeffektor, der ein erstes Endeffektor-Element mit einer ersten Oberfläche und ein zweites Endeffektor-Element mit einer zweiten Oberfläche, die so konfiguriert ist, dass sie mit der ersten Oberfläche zusammenwirkt; und ein Gelenk, das den Endeffektor mit der Welle verbindet, wobei das Gelenk es dem ersten Endeffektor-Element ermöglicht, sich um eine erste Achse und das zweite Endeffektorelement um eine zweite Achse zu drehen, wobei die erste und die zweite Achse quer zur Längsachse der Welle verlaufen; wobei, wenn der Endeffektor mit der Welle ausgerichtet ist und die erste und zweite Oberfläche miteinander verbunden sind, die Ausrichtung der ersten Oberfläche relativ zu der ersten Achse größer als Null Grad ist. Damit soll ein chirurgisches Instrument angegeben werden, das ein Höchstmaß an Präzision bietet und die Effizienz steigert.

Ein minimalinvasives Instrument ist aus der DE 10 2012 207 707 A1 bekannt, bei dem chirurgisches Material einfacher und schneller in den Patientenkörper eingeführt werden kann. Dafür ist zusätzlich ein manuelles Bedienelement vorgesehen, mit dem das Funktionselement, beispielsweise eine Zange, bedienbar bleibt, während das Instrument vom Operationsroboter abgekoppelt ist.

Die DE 10 2006 059 952 B3 beschreibt eine Roboterstruktur für die minimalinvasive Chirugie, bei der Greifkräfte mit einem Sensorelement direkt am Gelenk gemessen wird. Ebenso beschreibt die US 2009/0157092 A1 eine Kraftsensor-Vorrichtung, die einen Rohrabschnitt mit einer Vielzahl von radialen Rippen und einen Dehnungsmessstreifen, der über jeder der mehreren radialen Rippen angeordnet ist, ein proximales Ende des Rohrabschnitts, das funktionsfähig mit einem Schaft eines chirurgischen Instruments koppelt, das funktionsfähig mit einem Manipulatorarm eines chirurgischen Robotersystems koppelt, und ein distales Ende des Rohrteils, das proximal mit einem Handgelenk verbunden ist, das mit einem Endeffektor gekoppelt ist. Nachteilig ist dabei, dass das Sensorelement und die dafür erforderlichen Umlenkungen am Gelenk des Zangengreifers angeordnet sein müssen, womit diese Technik beim minimalinvasiven Eingriff beim Patienten mit in den Patientenkörper eingeführt werden muss. Entsprechend belastet diese Technik den möglichst kleinbauenden Gelenkkopf des Instruments, was sowohl deren Stabilität und/oder deren Ausmaße, die sterile Reinigungsfähigkeit und die Kosten bei dessen Ersatz beeinträchtigt.

Roboterassistierte laparoskopische Operationen sind in der jüngsten Entwicklung bei vielen chirurgischen Eingriffen eine bevorzugte Methode geworden. Dabei sind Nadelhalter als robotergeführte Werkzeuge für das Nähen von Gewebe in der allgemeinen Chirurgie bekannt. Diese robotergeführten Werkzeuge verwenden einen Zangengreifer, der um zwei Achsen kippbar ist. Die Aktuierung des Greifers und der Gelenke erfolgt über Seilzüge in Verbindung mit Umlenkrollen. Das laparoskopische Operationswerkzeug ist eine auswechselbare Komponente des Robotersystems. Der motorisierte Antrieb für den Seilzug des Werkzeugs ist dabei eine Komponente des Roboterarms. Das laparoskopische Operationswerkzeug ist eine über den Montagekopf am Roboterarm des Operationsroboters auswechselbare Komponente. Der Montagekopf ist die mechanisch/aktuatorische, lösbare Verbindung zum Roboterarm. Im Roboterarm sind Aktuatoren, beispielsweise E-Motoren verbaut, die über Zahnräder die Antriebskraft in den Montagekopf übertragen. Dort treiben die Zahnräder zugeordnete Seilzugrollen an, die das Antriebsmittel für das laparoskopische Operationswerkzeug, hier ausgebildet als Nadelhalter, betreiben. Somit ist der motorisierte Antrieb für die im Operationswerkzeug (Nadelhalter) angeordneten Seilzüge eine Komponente des Roboterarms. Das über den Montagekopf auswechselbare laparoskopische Operationswerkzeug, hier als Nadelhalter ausgebildet, ist dabei der Teil, der mit seinem Zangengreifer und dem Schaftelement zur Ausführung der minimalinvasiven Operation in den menschlichen Körper eingeführt wird. Da trotz aufwendiger Reinigungsmethoden kleinste Restanschmutzungen an dem laparoskopischen Operationswerkzeug nicht auszuschließen sind, werden zur Zeit diese Werkzeuge nach 10 Einsätzen entsorgt.

Die technischen Entwicklungen und die klinischen Vorteile in der laparoskopischen Chirurgie in den 1990er Jahren hatten bislang nur einen geringen Einfluss auf die Gefäßchirurgie. Im Gegensatz zur Laparoskopie arbeiten Robotik-Instrumente aufgrund der Endo-Wrist-Technologie grundsätzlich flexibler sind einfach zu bedienen und in allen Freiheitsgraden manipulierbar. Es ist somit möglich, topographisch komplexe chirurgische Eingriffe, die klassisch chirurgischen laparoskopischen Techniken nur schwer zugänglich sind, roboterassistiert präzise durchzuführen. In den Belangen der Gefäßchirurgie betrifft dies insbesondere das paraviszerale Segment im Hinblick auf renale und mesenteriale Bypässe. Diese Eingriffe sind aufgrund der schwierigen und traumatischen Exposition - vor allem bei adipösen Patienten - derzeit prinzipiell Domäne der endovaskulären Versorgung, wobei oftmals eine geringe Offenheitsrate der endovaskulären Gefäßversorgung in Kauf genommen wird.

In erster Linie liegt die Zurückhaltung der Laparoskopie in der Gefäßchirurgie an Schwierigkeiten beim Nähen der Gefäßanastomose verbunden mit einer dadurch resultierenden langen Klemmzeit der Gefäße. Derzeit repräsentieren roboterassistierte Aneurysma-Operationen und die Anlage aortofemoraler Bypässe die Standardoperationen in der Gefäßchirurgie. Inzwischen kann aufgrund hervorragender Single-Center Erfahrung mit den Vorteilen der roboterassistierten Gefäßchirurgie argumentiert werden, wie in P. Stádler, L. Dvorácek, P. Vitásek, P. Matous, - Robot assisted Aortic and Non-aortic Vascular Operations, Eur J Vasc Endovasc Surg (2016) 52, 22-28. Dies liegt neben einer langjährigen Expertise mit dem Robotik-System auch an der Etablierung eigener Techniken in Bezug auf Anastomosen Techniken und der Verwendung spezieller Materialien (z.B. gekürzte, reißfeste PTFE-Fäden, koppelbare Gefäßklemmen, etc.).

Aufgrund der minimal invasiven Traumatisierung profitieren die Patienten hauptsächlich durch kürzere Krankenhausliegedauer und eine frühere Rehabilitation sowie Rückkehr zu gewohnten Aktivitäten und Arbeitsleben. Das Verfahren ist prädestiniert bei adipösen Patienten; technisch anspruchsvolle Operationen mit dem Bedarf an einem übersichtlichen Operationssitus lassen sich nun ohne aufwendige Sperrersysteme, Lagerungsmodalitäten und Problemen bei der Wundheilung nach einer Laparotomie durchführen.

Der technische Fortschritt der Computer erhöht die Sicherheit und Präzision der Instrumentarführung im Körper des Patienten (Master and Slave-Prinzip). Darüber hinaus ist realisierbar, unter Verwendung einer Datenvernetzung ein virtuelles Mapping präoperativer Schnittbilddaten mit dem Operations-Situs zu koppeln und dem Operateur virtuelle on-demand Informationen während des Eingriffs zu vermitteln.

Die Abteilung der Gefäßchirurgie war bereits in der Entstehung des Kurt-Semm-Robotik-Zentrums 2016 am Universitätsklinikum Schleswig-Holstein (UKSH Kiel) integriert worden. Im Rahmen von experimentellen interdisziplinären Übungskursen an Körperspendern wurden erste gefäßchirurgische Erfahrungen gesammelt. Klinisch konnte eine Studie (n=18) roboterassistierter Patchanastomosen im iliaco-femoralen Bereich im Hinblick auf den Operationszugang, die Anastomosen Dauer und klinische Ergebnisse begonnen werden.

Die Schwierigkeit in der laparoskopisch bzw. roboterassistierten Gefäßchirurgie besteht in der Handhabung der Gefäßnaht. Die Besonderheit der Gefäßnaht liegt insbesondere an der manuell/technisch unterschiedlichen Durchführung im Vergleich zu anderen Fachdisziplinen, wie beispielsweise der Allgemein-/Viszeralchirurgie oder Urologie. In diesen Fachbereichen dient die Naht als Gewebeadaption und bedarf nur wenig Zugspannung bei der Anastomose. Im Vergleich dazu benötigt man bei der Gefäßrekonstruktion respektive der Aortenchirurgie deutlich höhere Kräfte für das Führen des Fadens. Dabei erfordert es Erfahrung mit der Handhabung des Nahtmaterials als auch das Gefühl für die Vulnerabilität von Gefäßen. Wenn der Faden zu stark geführt und unter Spannung gehalten wird, ist entweder ein Ein- oder Ausriss des Nahtmaterials aus dem Gefäß die Folge, oder der Faden selbst reißt unter dieser Belastung. Wird der Faden zu locker geführt, kommt es nach Freigabe des Blutflusses (z.B. von der Aorta in die anastomisierte Aortenprothese) zu Blutungen aus dem Anastomosenbereich, da die Dichtigkeit zwischen Gefäß und Prothese nicht gewährleitet ist - die Naht ist zu locker. In der offenen Chirurgie behilft man sich damit, dass die Assistenz das durchgezogene Fadenende stets auf Spannung hält, diese reicht entsprechend der Gewebekonsistenz von etwa 5 - 30 N. Diese Zugkraft wird solange beibehalten, bis der nächste Stich gesetzt ist, das lose Fadenende wird durchgezogen, entsprechend wird der angezogene Faden äquivalent dazu freigegeben. Das Resultat ist eine auf Spannung getätigte Gefäßnaht, welche das Gefäß und die Aortenprothese optimal approximiert und - sofern der entsprechende Stichkanalabstand eingehalten ist - eine Dichtigkeit gewährleistet.

Hier stellt sich die Problematik, dass bei laparoskopischen respektive roboterassistierten chirurgischen Eingriffen die Zugstärke am Faden nur sehr schlecht kontrolliert werden kann. Während man bei laparoskopischen Operationsverfahren eine haptische Rückmeldung der Zugstärke am Faden durch die manuelle Direktübertragung am Nadelhalter hat, ist dies auf Grund der ferngesteuerten Kraftbetätigung in modernen Robotiksystemen nicht möglich.

Als Resultat einer zu starken Zugkraft reißt der Faden und die Anastomose muss erneuert werden. Dieses findet sich sehr häufig bei monofilem Nahtmaterial (z.B. Prolene). Deshalb benutzt man für Gefäßnähte in der Regel PTFE-Fäden (Synonym: Teflon- Fäden), welche neben einer erhöhten Festigkeit auch eine gewisse Elastizität beinhalten. Jedoch besteht auch bei diesem Faden die gleiche Problematik, dass die am Nahtfaden wirkende Zugkraft nicht kontrollierbar ist und der Faden die Gefäßnaht nicht kontinuierlich unter einem gleichbleibenden Zug approximiert und folglich keine primär dichte Gefäßanastomose gewährleistet werden kann. Dieses chirurgische Nahtmaterial, beispielsweise GORE-TEX SUTURE SIZE CV-8 bis CV-0, hat im geknoteten Zustand Rissfestigkeiten von 0,3 bis 5,3 kg. Dies entspricht Zugkräften von 3 bis 53 N.

Aufgabe der Erindung ist es daher, einen Nadelhalter zum Halten eines Nahtfadens für roboterassistierte laparoskopische Operationen anzugeben, mit dem die jeweils wirkende Zugstärke des Nahtfadens kontrolliert werden kann.

Gelöst wird diese Aufgabe mit einem Nadelhalter gemäß Anspruch 1. Durch die Kraftdetektoren im Nadelhalter kann die Spannkraft des vom Zangengreifer gehaltenen Nahtfadens erfasst werden, sodass der Operateur an seiner Steuerkonsole während der roboterassistierten laparoskopischen Operation Rückmeldung über die jeweilig wirkende Nahtfaden-Zugkraft erhält. Dabei sind als Kraftdetektoren an jedem Seilzug Zugkraftsensoren angeordnet, die die am Seilzug wirkende Zugkraft messen. Die Rückmeldung kann über die Betätigungselemente an der Arztkonsole des Operationsroboters durch elektrisch aufgelastete "Gegenkräfte" oder durch Anzeigen der jeweils akut anstehenden Nahtfadenspannung als absolute Größe oder als optisch dargestellter Zielwert zwischen einer Untergrenze und einer Obergrenze dargestellt werden. Somit ist der Operateur von seiner Steuerkonsole aus präzise in der Lage, die richtige Zugspannung auf den Nahtfaden auszuüben, um weder das zu nähende Gewebe und/oder den Nahtfaden zu überlasten, noch den Faden zu locker zu führen, um Blutungen aus dem Anastomosenbereich zu verhindern. Beispielsweise kann die Nahtfadenspannung auf einen Wert von 5 bis 30 N gehalten werden.

Eine Erfassung der Spannkraft des Nahtfadens sollte stets möglichst nahe am Angriffspunkt der Kraft erfolgen. Andererseits ist eine Messung direkt am Zangengreifer bzw. dem Gelenkkopf kaum möglich, da das zur minimalinvasiven Chirurgie im menschlichen Körper vorgesehene Operationswerkzeug sehr kleinbauend, mechanisch empfindlich und auch aus hygienischen Gründen möglichst wenig bewegliche Elemente und/oder zusätzliche Sensoren, Kabel, etc. aufweisen sollte. Insoweit ist eine Messung der Seilzugspannung an den Seilzügen innerhalb des Operationswerkzeugs, also eine indrekte Erfassung der Kräfte durch Bestimmung der Spannkraft in den Seilzügen zur Betätigung des Gelenkkopfes für den Zangengreifer vorzusehen. Damit ist eine indirekte Messung der auf den Nahtfaden wirkenden Zugkraft am im Zangengreifer des Nadelhalters gehaltenen Nahtfadens möglich.

Dabei muss bedacht werden, dass der Angriffspunkt der Fadenspannung am Ende des Zangengreifers erfolgt. Sollte dieser beispielsweise um 90° zur Längsachse des Operationswerkzeugs angestellt sein, wirkt ein entsprechendes Drehmoment. Dieses Drehmoment spannt nun den Seilzug, welcher zur Aktuierung des Zangengreifers um diese Schwenkachse genutzt wird. Wird beispielsweise ein GORE-TEX Nahtfaden CV-6 mit einer Rissspannung von F1 = 6,5 N verwendet, würde das dafür erforderliche Drehmoment bei der vorangehend dargestellten Abwinkelung der Greifzange mit M = F1 * L = Rissspannung im Faden * Länge des Greifers (z.B. 15 mm) = 100 N * mm ergeben. Bei einem Radius der an dieser Schwenkachse angesetzten Umlenkrolle für den betreffenden Seilzug von r = 3 mm ergibt sich entsprechend dem Gleichgewicht der wirkenden Drehmomente auf den Seilzug eine 5-fach größere Spannung mit F2 = M / r, also F2 = 33 N. Diese Kraft bzw. Seilspannung ist entsprechend mit dem Kraftsensor im Montagekopf des Werkzeugs (Nadelhalter) als auf keinen Fall zu überschreitende Grenzkraft zu detektieren.

Dabei hat der Zugkraftsensor bevorzugt drei hintereinander angeordnete, drehbare Rollen, über die der Seilzug wechselseitig geführt ist, wobei zwei äußere Rollen ortsfest im Nadelhalter gelagert sind und eine mittlere Rolle in ihrer Radialebene senkrecht zum Seilzug federnd beweglich ist, wobei die am Seilzug wirkende Zugkraft eine Auslenkung der mittleren Rolle mit einer resultierenden, zu messenden Kraft bewirkt, die proportional zur am Seilzug wirkenden Zugkraft ist. Dabei ist dieser Zugkraftsensor im Montagekopf angeordnet, da dort entsprechender Bauraum zur Verfügung steht und das proximale Ende des Operationswerkzeugs mit seinem Montagekopf möglichst weit von der Operationsstelle entfernt bleibt und mithin nicht oder nur kaum mit Körperflüssigkeiten belastet und somit hygienisch nicht oder zumindest nicht stark belastet ist.

Dadurch, dass eine flache Knickung von 1° bis 15° des Seilzuges um die mittlere Rolle vorgesehen ist, wird die an der mittleren Rolle gegen die Federkraft wirkende, zu messende Kraft gegenüber der in Richtung des Seilzuges wirkenden Kraft erheblich reduziert, so dass hier beispielsweise relativ geringe Messbereiche von beispielsweise 0 bis 5 N zu erfassen sind. Derartige Kraftbereiche können mit Miniatur-Biegebalken-Zug-Druckkraftsensoren, wie beispielsweise Typ 8510 von Burster Präzisionsmesstechnik GmbH & Co, KG, Talstraße 1-5, 76593 Gernsbach gemessen werden. Dabei variieren die zu messenden Kräfte am Seilzug beispielsweise im Bereich von 20 bis 24 N.

Nachfolgend wird der Anmeldungsgegenstand anhand der beiliegenden Zeichnungen detailliert beschrieben.

Darin zeigt:
- Fig. 1: ein Ausführungsbeispiel eines laparoskopischen Operationswerkzeugs als Nadelhalter und
- Fig. 2: eine teils geschnittene Detaildarstellung des Nadelhalters gemäß Fig. 1 mit einem Zugkraftsensor zur Kraftdetektion an einem Seilzug.

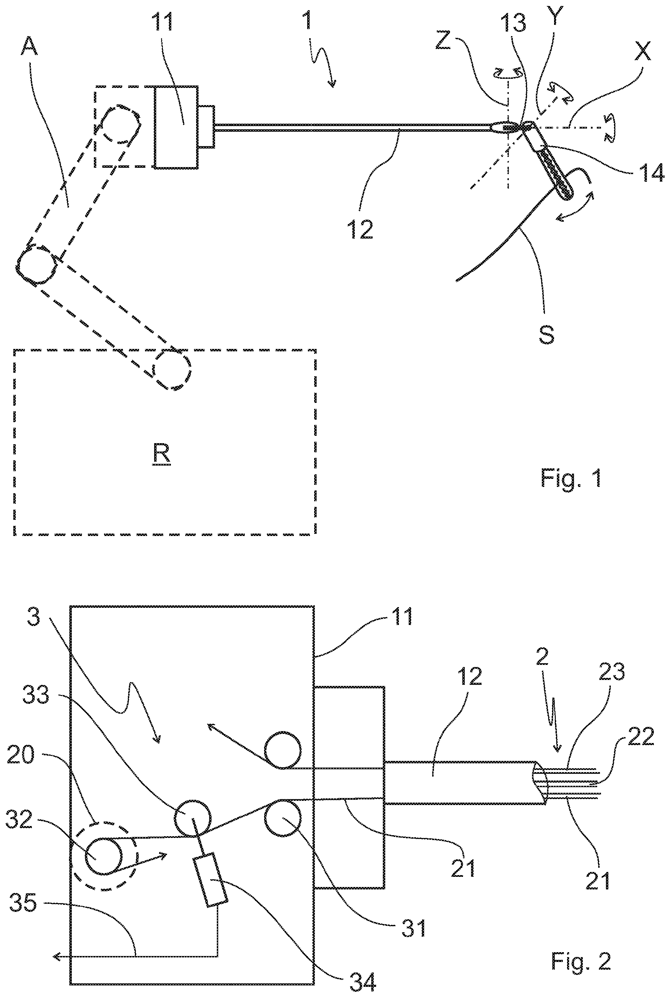

In Fig. 1 ist schematisch ein Operationsroboter R mit einem Roboterarm A gestrichelt dargestellt. Am freien Ende des Roboterarms A ist ein laparoskopisches Operationswerkzeug in Form eines Nadelhalters 1 über einen Montagekopf 11 angekoppelt. Der Nadelhalter 1 weist ferner ein langgestrecktes Schaftelement 12 auf, das vom Montagekopf 11 bis zu einem Gelenkkopf 13 führt. Am Gelenkkopf 13 ist ein Zangengreifer 14 angesetzt. Der Gelenkkopf 13 erlaubt Verschwenkungen um zwei zueinander senkrecht orientierte Achsen, die im Wesentlichen senkrecht zur Längsachse des Schaftelementes 12 orientiert sind. Ferner ist das Schaftelement 12 um seine Längsachse drehbar im Montagekopf 11 gehaltert. Entsprechend kann der Zangengreifer 14 vielfältige Bewegungen ausführen.

In Fig. 2 ist in einer Detaildarstellung der Nadelhalter 1, nämlich der Montagekopf 11 sowie das daran anschließende Schaftelement 12 dargestellt. Im Schaftelement 12 sind in seinem hohlen Innenraum drei Antriebsmittel in Form von Seilzügen angedeutet. Ein erster Seilzug 21 bewirkt über eine nicht dargestellte Umlenkrolle am Gelenkkopf 13 ein Verschwenken um eine erste Schwenkachse Y (strichpunktierte Linie in Fig. 1). Ferner ist im Innenraum des Schaftelementes 12 ein zweites Antriebsmittel als zweiter Seilzug 22 ausgebildet, der über eine weitere nicht dargestellte Umlenkrolle auf die zweite Schwenkachse Z (strickpunktiert in Fig. 1) wirkt. Ein drittes Antriebsmittel als dritter Seilzug 23 in dem Schaftelement 12 führt zum Zangengreifer 14, mit dem der Zangengreifer geöffnet und geschlosen werden kann.

Im Montagekopf 11 ist für den ersten Seilzug 21 ein Zugkraftsensor 3 im Detail dargestellt (Fig. 2). Der Zugkraftsensor 3 besteht aus einer ersten äußeren Rolle 31 und einer zweiten äußeren Rolle 32, die beide ortsfest in dem Montagekopf 11 angeordnet sind. Zwischen der ersten äußeren Rolle 31 und der zweiten äußeren Rolle 32 ist eine mittlere Rolle 33 angeordnet, die in ihrer Radialebene senkrecht zum Seilzug federnd beweglich angeordnet ist. Daran ist ein Zugkraftmessgerät 34 befestigt, welches die Seilspannung des ersten Seilzuges 21 aufgrund der geringen Knickung des Seilzuges zwischen erster äußerer Rolle 31, mittlerer Rolle 33 und zweiter äußeren Rolle 32 messen kann. Die am Zugkraftmessgerät 34 ermittelten Messsignale werden über Wirkleitung 35 im Montagekopf 11 über den Roboterarm A dem Operationsroboter R übermittelt.

Ein ähnlicher Aufbau ist auch für die weiteren Antriebsmittel, nämlich den zweiten Seilzug 22 und optional auch den dritten Seilzug 23 mit entsprechenden Zugkraftsensoren 3 vorgesehen, die hier jedoch nicht einzeln dargestellt sind. Nach dem Koppeln des Montagekopfes 11 an den Roboterarm A des Operationsroboters R werden Antriebskräfte von Aktuatoren, insbesondere Elektromotoren innerhalb des Roboterarms A über Zahnräder und entsprechende Koppelmechanismen auf zugeordnete Zahnräder 20 im Montagekopf 11 übertragen, die mit dem jeweiligen Antriebsmittel, hier erster Seilzug 21 wirkverbunden sind. Entsprechend wird die Antriebskraft auf den Nadelhalter 1 in bekannter Weise übertragen. Durch die intergrierten Zugkraftsensoren 3 und deren Rückmeldung an den Operationsroboter R wird nunmehr jedoch die Zugkraft im zugeordneten Seilzug, hier erster Seilzug 21, erfasst. Durch diese Bestimmung der Spannkraft in dem jeweiligen Seilzug können die am Zangengreifer 14 wirkenden Kräfte indirekt erfasst werden. Entsprechend kann die auf einen im Zangengreifer 14 gehaltenen Nahtfaden wirkende Zugkraft erfasst und eine nicht zu überschreitende Maximalbelastung überwacht werden. Dabei kann der Operationsroboter R dem Operateur ein akkustisches und/oder visuelles Warnsignal geben, wonach der Operateur die empfohlene Zugkraft für beispielsweise eine Gefäßnaht erreichen kann.

Somit ist im Nadelhalter 1, der bei roboterassistierten laparoskopischen Operationen benutzt wird, ein Zugkraftsensor 3 als elektronisches Gerät integriert. Dabei wird die Fadenspannung bzw. Kraft am Greifer über die Spannung in den Seilzügen des Werkzeugs erfasst. Zwei der drei Seilzüge des Werkzeugs dienen der Aktuierung der beiden Gelenke. Es wird somit eine Vorrichtung zur Erfassung der Seilspannung vorgeschlagen, welche die Integrität des Zugseiles und damit die Zuverlässigkeit des Systems nicht beeinflusst.

### Bezugszeichenliste

- 1: Laparoskopisches Operationswerkzeug, Nadelhalter
- 11: Montagekopf
- 12: Schaftelement
- 13: Gelenkkopf
- 14: Zangengreifer

- 2: Antriebsmittel
- 20: Zahnrad
- 21: erstes Antriebsmittel, erster Seilzug
- 22: zweites Antriebsmittel, zweiter Seilzug
- 23: drittes Antriebsmittel, dritter Seilzug

- 3: Zugkraftsensor, Kraftdetektor
- 31: erste äußere Rolle
- 32: zweite äußere Rolle
- 33: mittlere Rolle
- 34: Zugkraftmessgerät
- 35: Wirkleitung

- A: Roboterarm
- R: Operationsroboter
- S: Nahtfaden
- X: Längsachse
- Y: erste Schwenkachse
- Z: zweite Schwenkachse

## Patentansprüche

1. Nadelhalter (1) zum Halten eines Nahtfadens (S) für roboterassistierte laparoskopische Operationen, die mittels eines Operationsroboters (R) mit wenigstens einem Roboterarm (A) durchgeführt werden, mit einem Montagekopf (11) zum Ankoppeln an den Roboterarm (A), einem Schaftelement (12), einem am distalen Ende des Schaftelements (12) angeordneten Gelenkkopf (13) und einem am Gelenkkopf (13) um wenigstens zwei Schwenkachsen (Y, Z) mittels eines ersten und zweiten Antriebsmittels (21, 22) kippbar angeordneten Zangengreifer (14), wobei die Antriebsmittel über Umlenkrollen geführte Seilzüge (21, 22) sind, wobei Kraftdetektoren (3) im Nadelhalter (1) angeordnet sind, die die Spannkraft des vom Zangengreifer (14) gehaltenen Nahtfadens (S) erfassen, **dadurch gekennzeichnet, dass** als Kraftdetektoren an jedem Seilzug (21, 22) Zugkraftsensoren (3) angeordnet sind, die die am Seilzug (21, 22) wirkende Zugkraft messen, wobei jeder Zugkraftsensor (3) drei hintereinander angeordnete, drehbare Rollen (31, 32, 33) hat, über die der Seilzug (21, 22) wechselseitig geführt ist, wobei zwei äußere Rollen (31, 32) ortsfest im Nadelhalter gelagert sind und eine mittlere Rolle (33) in ihrer Radialebene senkrecht zum Seilzug (21, 22) federnd beweglich ist, wobei die am Seilzug wirkende Zugkraft eine Auslenkung der mittleren Rolle (33) mit einer resultierenden, zu messenden Kraft bewirkt, die proportional zur am Seilzug (21, 22) wirkenden Zugkraft ist, und der/die Zugkraftsensor(en) (3) im Montagekopf (11) angeordnet sind.

2. Nadelhalter nach Anspruch 1, **dadurch gekennzeichnet, dass** eine flache Knickung von 1° bis 15° des Seilzuges (21, 22) um die mittlere Rolle (33) vorgesehen ist.

3. Nadelhalter nach Anspruch 1 oder 2, **dadurch gekennzeichnet, dass** der Zangengreifer (14) über ein drittes Antriebsmittel (23) antreibbar ausgebildet ist, wobei das dritte Antriebsmittel ebenfalls ein über Umlenkrollen geführter dritter Seilzug (23) ist.

## Claims

1. Needle holder (1) for holding a suture thread (S) for robot-assisted laparoscopic surgery performed by means of a surgical robot (R) with at least one robot arm (A), comprising a mounting head (11) for coupling to the robot arm (A), a shaft element (12), a joint head (13) arranged at the distal end of the shaft element (12) and a pair of forceps grippers (14) arranged on the joint head (13) so as to be tiltable about at least two pivot axes (Y, Z) by means of a first and second drive means (21, 22), wherein the drive means are cable pulls (21, 22) guided over deflection rollers, force detectors (3) being arranged in the needle holder (1) which detect the tension force of the suture thread (S) held by the pair of forceps grippers (14), **characterised in that** tensile force sensors (3) are arranged as force detectors on each cable pull (21, 22), which measure the tensile force acting on the cable pull (21, 22), wherein each tensile force sensor (3) has three rotatable rollers (31, 32, 33) arranged one behind the other, over which the cable pull (21, 22) is guided alternately, wherein two outer rollers (31, 32) are mounted fixedly in the needle holder and a middle roller (33) is resiliently movable in its radial plane perpendicular to the cable pull (21, 22), wherein the tensile force acting on the cable pull causes a deflection of the middle roller (33) with a resulting force to be measured which is proportional to the tensile force acting on the cable pull (21, 22), and the tension force sensor(s) (3) are arranged in the mounting head (11).

2. Needle holder according to claim 1, **characterised in that** a flat bend of 1° to 15° of the cable pull (21, 22) around the middle roller (33) is provided.

3. Needle holder according to claim 1 or 2, **characterised in that** the pair of forceps grippers (14) is designed to be driven by a third drive means (23), wherein the third drive means is a third cable pull (23) also guided via deflection rollers.

## Revendications

1. Porte-aiguille (1) pour tenir un fil de suture (S) pour des opérations laparoscopiques assistées par robot qui sont réalisées à l'aide d'un robot chirurgical (R) avec au moins un bras robotisé (A), comprenant une tête de montage (11) pour le coupler au bras robotisé (A), un élément de tige (12), une tête d'articulation (13) disposée à l'extrémité distale de l'élément de tige (12) et un préhenseur à pinces (14) articulé au niveau de la tête d'articulation (13) autour d'au moins deux axes de pivotement (Y, Z) au moyen d'un premier et d'un second moyen d'entraînement (21, 22), les moyens d'entraînement étant des câbles (21, 22) guidés par des rouleaux de guidage, des détecteurs de force (3) étant disposés dans le porte-aiguille (1) pour détecter la force de tension du fil de suture (S) tenu par le préhenseur à pinces (14), **caractérisé en ce que,** en tant que détecteurs de force, des capteurs de force de traction (3) sont disposés sur chaque câble (21, 22) pour mesurer la force de traction exercée sur le câble (21, 22), chaque capteur de force de traction (3) comprenant trois rouleaux rotatifs (31, 32, 33) disposés les uns derrière les autres, par l'intermédiaire desquels le câble (21, 22) est guidé de façon alternée, deux rouleaux extérieurs (31, 32) étant montés de manière fixe dans le porte-aiguille et un rouleau central (33) étant mobile de manière élastique dans son plan radial perpendiculaire au câble (21, 22), la force de traction exercée sur le câble (21, 22) provoquant un déplacement du rouleau central (33) avec une force résultante mesurable qui est proportionnelle à la force de traction exercée sur le câble (21, 22), et le(s) capteur(s) de force de traction (3) étant disposé(s) dans la tête de montage (11).

2. Porte-aiguille selon la revendication 1, **caractérisé en ce qu'**il est prévu une courbure plate de 1° à 15° du câble (21, 22) autour du rouleau central (33).

3. Porte-aiguille selon la revendication 1 ou 2, **caractérisé en ce que** le préhenseur à pinces (14) est conçu pour pouvoir être entraîné par un troisième moyen d'entraînement (23), le troisième moyen d'entraînement étant également un troisième câble (23) guidé par des rouleaux de guidage.
